# EUROPEAN PATENT SPECIFICATION

(11) **EP 4 279 477 B1**
(45) Date of publication and mention of the grant of the patent: **23.10.2024**
(21) Application number: 21923690.8
(22) Date of filing: 03.02.2021
(51) Int. Cl.: C07C 51/09, C07C 67/08, C07C 59/06, C07C 69/145, C07C 69/675, C07C 51/377, C07C 67/31

(54) **METHOD FOR PREPARING GLYCOLIC ACID AND METHYL GLYCOLATE THROUGH HYDROLYSIS OF METHYL METHOXYACETATE AND METHOXYACETIC ACID**
VERFAHREN ZUR HERSTELLUNG VON GLYKOLSÄURE UND METHYLGLYKOLAT DURCH HYDROLYSE VON METHYLMETHOXYACETAT UND METHOXYESSIGSÄURE
PROCÉDÉ DE PRÉPARATION D'ACIDE GLYCOLIQUE ET DE GLYCOLATE DE MÉTHYLE PAR HYDROLYSE DE MÉTHOXYACÉTATE DE MÉTHYLE ET D'ACIDE MÉTHOXYACÉTIQUE

(43) Date of publication of application: 22.11.2023
(73) Proprietor: Dalian Institute of Chemical Physics, Chinese Academy of Sciences, Dalian, Liaoning 116023 (CN)
(72) Inventor: NI, Youming, Dalian, Liaoning 116023 (CN); ZHU, Wenliang, Dalian, Liaoning 116023 (CN); LIU, Zhongmin, Dalian, Liaoning 116023 (CN)
(74) Representative: Bayramoglu et al.
(86) International application number: PCT/CN2021/075023
(87) International publication number: WO 2022/165662

(56) References cited:
- WO-A1-2013/148482
- CN-A- 1 180 067
- CN-A- 106 554 250
- CN-A- 107 602 388
- DE-B- 1 130 431
- JP-A- 2003 300 926

## Description

### TECHNICAL FIELD

The present application relates to a method for preparing glycolic acid and methyl glycolate through hydrolysis of methyl methoxyacetate and methoxyacetic acid, and belongs to the technical field of preparation of chemical products.

### BACKGROUND

Glycolic acid, also known as hydroxyacetic acid, is the simplest α-hydroxycarboxylic acid compound. Methyl glycolate can be hydrogenated to produce ethylene glycol (EG), and can also be easily hydrolyzed under mild conditions to produce glycolic acid. Because the molecular structure of glycolic acid includes both hydroxyl and carboxyl, glycolic acid can undergo self-polymerization to produce polyglycolic acid (PGA). PGA has not only excellent biocompatibility, but also safe biodegradability. Therefore, PGA is widely used not only for medical surgical sutures, drug sustained-release materials, and degradable human tissue scaffolds, but also in production of common plastic products. Conventional non-degradable plastic products have caused heavy environmental pollution, and biodegradable PGA plastics are expected to solve this problem. Glycolic acid can also undergo copolymerization with monomers such as lactic acid and hydroxypropionic acid to produce a polymer material with excellent performance and extensive applications. In addition, glycolic acid is an excellent chemical cleaning agent and cosmetic raw material.

Preparation methods of glycolic acid mainly include a chloroacetic acid hydrolysis method, a formaldehyde carbonylation method, and an oxalate hydrogenation/hydrolysis method. In the chloroacetic acid hydrolysis method, the preparation process of the raw material chloroacetic acid causes heavy pollution, and the hydrolysis process of chloroacetic acid leads to a large amount of waste salt, heavy pollution, and poor quality product. Thus, the chloroacetic acid hydrolysis method has been basically eliminated. The formaldehyde carbonylation method involves cheap and easily-available raw materials, but needs to be implemented under conditions such as a high temperature, a high pressure, a strong liquid acid, and an organic solvent. In addition, in the formaldehyde carbonylation method, the device is easily corroded, and the product purification is difficult, resulting in a high industrial production cost. In the oxalate hydrogenation/hydrolysis method, an oxalate is partially hydrogenated to produce methyl glycolate, and then methyl glycolate is hydrolyzed to produce glycolic acid. However, the catalyst for partial hydrogenation of the oxalate is not mature and has low conversion efficiency and poor stability; and the oxalate production process is cumbersome and costly. These problems seriously restrict the development of the oxalate hydrogenation/hydrolysis method.

In recent years, a dimethoxymethane (DMM) carbonylation reaction to prepare methyl methoxyacetate has received widespread attention. This reaction is based on a molecular sieve catalyst, can be conducted at a low reaction temperature, and has high atomic economy. The raw material DMM can be produced by a very mature industrialized technology with high efficiency, and thus is cheap.

However, in the prior art, methyl methoxyacetate produced from DMM carbonylation has not been applied to preparation of glycolic acid and methyl glycolate. In addition, methoxyacetic acid resulting from hydrolysis of an ester bond of methyl methoxyacetate is relatively less used, resulting in waste of the raw material. CN 107602388 discloses the conversion of methyl methoxy acetate to methyl glycolate and glycolic acid using hydroiodic acid as a catalyst.

### SUMMARY

According to an aspect of the present application, a method for preparing glycolic acid and methyl glycolate through hydrolysis of methyl methoxyacetate and methoxyacetic acid is provided. The method of the present application is particularly suitable for carbonylation of DMM produced in the coal chemical industry to produce methyl methoxyacetate and then hydrolysis to produce glycolic acid and methyl glycolate. This reaction process can be used in combination with DMM carbonylation for preparing methyl methoxyacetate to allow efficient, environmentally-friendly, and economical conversion of DMM (a platform chemical in the coal chemical industry) into glycolic acid and methyl glycolate, which expands the use of methyl methoxyacetate and methoxyacetic acid and brings beneficial effects.

A method for preparing glycolic acid and methyl glycolate through hydrolysis of methyl methoxyacetate and methoxyacetic acid is provided, including: allowing raw materials including the methyl methoxyacetate, the methoxyacetic acid, and water to contact and react with a catalyst to produce the glycolic acid and the methyl glycolate,
where the catalyst is a solid acid catalyst;
the solid acid catalyst is at least one selected from the group consisting of an acidic molecular sieve catalyst, an acidic resin catalyst, and an acidic alumina catalyst; and
the acidic molecular sieve catalyst includes an acidic molecular sieve.

In recent years, the DMM carbonylation reaction to prepare methyl methoxyacetate has received widespread attention. This reaction is based on a molecular sieve catalyst, can be conducted at a low reaction temperature, and has high atomic economy. The raw material DMM can be produced by a very mature industrialized technology with high efficiency, and thus is cheap. In the present application, glycolic acid, methyl glycolate, and methoxyacetic acid can be obtained through hydrolysis of ether and ester bonds in the methyl methoxyacetate. Because methoxyacetic acid is relatively less used and can be returned to a reactor to undergo co-hydrolysis with methyl methoxyacetate, the method of the present application becomes an environmentally-friendly and economical production path for glycolic acid and methyl glycolate.

Optionally, the acidic molecular sieve is at least one selected from the group consisting of an acidic MFI-structured molecular sieve, an acidic FAU-structured molecular sieve, an acidic FER-structured molecular sieve, an acidic BEA-structured molecular sieve, an acidic MOR-structured molecular sieve, and an acidic MWW-structured molecular sieve.

Preferably, the acidic molecular sieve is any one selected from the group consisting of an acidic MFI-structured molecular sieve and an acidic FER-structured molecular sieve.

Optionally, the acidic molecular sieve is at least one selected from the group consisting of an acidic ZSM-5 molecular sieve, an acidic Y molecular sieve, an acidic ZSM-35 molecular sieve, an acidic β molecular sieve, an acidic MOR molecular sieve, and an acidic MCM-22 molecular sieve.

Preferably, the acidic molecular sieve is at least one selected from the group consisting of an acidic ZSM-5 molecular sieve and an acidic ZSM-35 molecular sieve.

Optionally, the acidic molecular sieve is at least one selected from the group consisting of a hydrogen-type ZSM-5 molecular sieve, a hydrogen-type Y molecular sieve, a hydrogen-type ZSM-35 molecular sieve, a hydrogen-type β molecular sieve, a hydrogen-type MOR molecular sieve, and a hydrogen-type MCM-22 molecular sieve.

Preferably, the acidic molecular sieve is at least one selected from the group consisting of a hydrogen-type ZSM-5 molecular sieve and a hydrogen-type ZSM-35 molecular sieve.

Optionally, a Si/Al atom ratio of the acidic molecular sieve is 3 to 500.

Specifically, an upper limit of the Si/Al atom ratio of the acidic molecular sieve is selected from the group consisting of 20, 10, 50, 100, and 500; and a lower limit of the Si/Al atom ratio of the acidic molecular sieve is selected from the group consisting of 3, 10, 20, 50, and 100.

Preferably, a Si/Al atom ratio of the acidic molecular sieve is 20 to 500.

Optionally, a content of the acidic molecular sieve in the acidic molecular sieve catalyst is 50 wt% to 100 wt%.

Optionally, the acidic molecular sieve catalyst further includes a forming agent; the forming agent is an oxide; and the oxide is one selected from the group consisting of alumina and silicon oxide.

Optionally, a content of the forming agent in the acidic molecular sieve catalyst is m, and 0 < m ≤ 50 wt%.

Optionally, the acidic molecular sieve catalyst is a fresh acidic molecular sieve catalyst and/or a regenerated acidic molecular sieve catalyst; and the fresh acidic molecular sieve catalyst is an unused acidic molecular sieve catalyst.

Optionally, a regeneration method of the acidic molecular sieve catalyst includes: treating an inactivated acidic molecular sieve catalyst with an oxygen-containing regeneration gas at 400°C to 800°C for 0.5 h to 24 h to obtain the regenerated acidic molecular sieve catalyst,
where a volume fraction of oxygen in the oxygen-containing regeneration gas is 0.5% to 50%.

Optionally, the acidic resin catalyst is any strongly-acidic cation exchange resin.

Optionally, a skeleton structure in the strongly-acidic cation exchange resin is a copolymer of styrene and divinylbenzene (DVB); and
an acidic group in the strongly-acidic cation exchange resin is a sulfonic acid group.

Optionally, the acidic alumina catalyst is γ-alumina.

Optionally, conditions of the reaction are as follows:
a reaction temperature is 60°C to 260°C;
a reaction pressure is 0.1 MPa to 10 MPa; and
in the raw materials, a ratio of a total mole number of the methyl methoxyacetate and the methoxyacetic acid to a mole number of the water is 1:2 to 1:20 and
a ratio relationship between the methyl methoxyacetate and the methoxyacetic acid is not limited.

Specifically, an upper limit of the reaction temperature is selected from the group consisting of 130°C, 160°C, 200°C, and 260°C; and a lower limit of the reaction temperature is selected from the group consisting of 60°C, 130°C, 160°C, and 200°C.

An upper limit of the reaction pressure is selected from the group consisting of 0.3 MPa, 1 MPa, 5 MPa, and 10 MPa; and a lower limit of the reaction pressure is selected from the group consisting of 0.1 MPa, 0.3 MPa, 1 MPa, and 5 MPa.

An upper limit of the ratio of a total mole number of the methyl methoxyacetate and the methoxyacetic acid to a mole number of the water is selected from the group consisting of 1:3, 1:6, 1:8, 1:10, 1:15, and 1:20; and a lower limit of the ratio of a total mole number of the methyl methoxyacetate and the methoxyacetic acid to a mole number of the water is selected from the group consisting of 1:2, 1:3, 1:6, 1:8, 1:10, and 1:15.

Preferably, the conditions of the reaction are as follows:
the reaction temperature is 130°C to 200°C;
the reaction pressure is 0.1 MPa to 0.3 MPa; and
in the raw materials, a ratio of a total mole number of the methyl methoxyacetate and the methoxyacetic acid to a mole number of the water is 1:3 to 1:8 and
a molar ratio of the methyl methoxyacetate to the methoxyacetic acid is 4:1 to 9:1.

Specifically, an upper limit of the molar ratio of the methyl methoxyacetate to the methoxyacetic acid is selected from the group consisting of 5:1 and 9:1; and a lower limit of the molar ratio of the methyl methoxyacetate to the methoxyacetic acid is selected from the group consisting of 4:1 and 5:1.

Possible beneficial effects of the present application:
1) The method for preparing glycolic acid and methyl glycolate in the present application can be implemented by a traditional fixed-bed reactor, tank reactor, or catalytic distillation reactor under an atmospheric pressure, which is very suitable for continuous production.
2) When used in combination with methanol and formaldehyde condensation to prepare DMM and DMM carbonylation to prepare methyl methoxyacetate, the method in the present application can allow efficient, environmentally-friendly, and economical conversion of methanol (a platform chemical in the coal chemical industry) into glycolic acid and methyl glycolate.

### BRIEF DESCRIPTION OF THE DRAWINGS

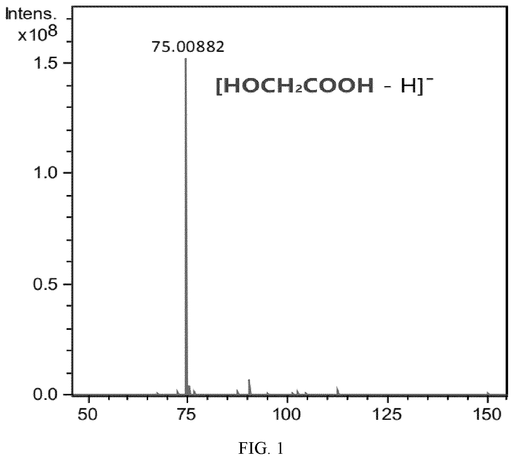

FIG. 1 is a negative-ion mass spectrum of glycolic acid among reaction products analyzed by liquid chromatography-mass spectrometry in Example 1 of the present application.

### DETAILED DESCRIPTION OF THE EMBODIMENTS

The present application will be described in detail below with reference to embodiments, but the present application is not limited to these embodiments.

Possible embodiments are described below.

According to the problems faced by the existing production technologies of glycolic acid and methyl glycolate, the present application discloses a method for preparing glycolic acid and methyl glycolate through hydrolysis of methyl methoxyacetate and methoxyacetic acid. The method of the present application is particularly suitable for carbonylation of DMM produced in the coal chemical industry to produce methyl methoxyacetate and then hydrolysis to produce glycolic acid and methyl glycolate.

Specifically, the present application provides a method for preparing glycolic acid and methyl glycolate through hydrolysis of methyl methoxyacetate and methoxyacetic acid, where raw materials of methyl methoxyacetate, methoxyacetic acid, and water are allowed to pass through a reaction zone loaded with a catalyst, such that the raw materials undergo a reaction under specified conditions to produce glycolic acid and methyl glycolate.

The catalyst is one or a mixture of two or more selected from the group consisting of a solid acid catalyst, a liquid acid catalyst, a solid base catalyst, and a liquid base catalyst.

The reaction zone includes a single fixed-bed reactor, or a plurality of fixed-bed reactors connected in series and/or parallel; or a single tank reactor, or a plurality of tank reactors connected in series and/or parallel; or a single catalytic distillation reactor, or a plurality of catalytic distillation reactors connected in series and/or parallel.

Conditions of the reaction are as follows: a reaction temperature is 60°C to 260°C; in the raw materials, a molar ratio of methyl methoxyacetate + methoxyacetic acid to water is 1:20 to 1:2, and a molar ratio of methyl methoxyacetate to methoxyacetic acid is any ratio; and a reaction pressure is 0.1 MPa to 10 MPa.

A reaction equation for hydrolysis of methyl methoxyacetate is as follows:

CH₃OCH₂COOCH₃ + 2 H₂O = 2 CH₃OH + HOCH₂COOH (1).

There are also two partial hydrolysis reactions as follows:

CH₃OCH₂COOCH₃ + H₂O = CH₃OH +HOCH₂COOCH₃ (2)

and

CH₃OCH₂COOCH₃ + H₂O = CH₃OH + CH₃OCH₂COOH (3).

Methoxyacetic acid in reaction (3) is further hydrolyzed under the same catalyst and reaction conditions to produce glycolic acid:

CH₃OCH₂COOH + H₂O = CH₃OH + HOCH₂COOH (4).

Reactions (1) to (4) are reversible reactions. In addition, methanol resulting from hydrolysis can also be partially dehydrated to produce dimethyl ether (DME).

The solid acid catalyst is one or a mixture of two or more selected from the group consisting of an acidic molecular sieve catalyst, an acidic resin catalyst, and an acidic alumina catalyst.

In addition to an acidic molecular sieve, the acidic molecular sieve catalyst further includes a catalyst forming agent with a weight percentage of 0% to 50%; and the catalyst forming agent is one selected from the group consisting of alumina and silicon oxide.

The acidic molecular sieve catalyst is a fresh acidic molecular sieve catalyst and/or a regenerated acidic molecular sieve catalyst.

A preparation method of the regenerated acidic molecular sieve catalyst includes: using a gas including oxygen in a volume fraction of 0.5% to 50% to treat an inactivated acidic molecular sieve catalyst obtained after hydrolysis of methyl methoxyacetate and methoxyacetic acid at 400°C to 800°C for 0.5 h to 24 h.

The acidic resin catalyst is a strongly-acidic cation exchange resin.

A skeleton structure in the strongly-acidic cation exchange resin is a copolymer of styrene and DVB; and an acidic group in the strongly-acidic cation exchange resin is a sulfonic acid group.

The acidic alumina catalyst is γ-alumina.

The γ-alumina is prepared by calcining an SB powder at 400°C to 800°C.

The liquid acid catalyst is an acidic liquid.

The liquid acid catalyst is one or more selected from the group consisting of sulfuric acid, hydrochloric acid, nitric acid, and phosphoric acid.

A concentration of H⁺ in the liquid acid catalyst is 0.01 mol/L to 10 mol/L.

The solid base catalyst is one or more selected from the group consisting of hydrotalcite, an anion exchange resin, and hydroxyapatite.

A composition of the hydrotalcite can be expressed as [Mg₁₋ₓAlₓ(OH)₂]^{x+}[CO₃²⁻]_{x/2}·n H₂O, where x is 0.1 to 0.34 and n is an integer of 0 to 4; and Mg can undergo isomorphous replacement by Zn, Fe, Co, Ni, and Cu and Al can be replaced by Cr, Fe, and In.

A composition of the hydroxyapatite can be expressed as Ca₁₀-ₓ(HPO₄)ₓ(PO₄)₆₋ₓ(OH)₂₋ₓ, where x is 0 to 1.

The liquid base catalyst is an alkaline liquid.

The liquid base catalyst is one or more selected from the group consisting of a sodium hydroxide aqueous solution, a potassium hydroxide aqueous solution, a calcium hydroxide aqueous solution, and a magnesium hydroxide aqueous solution.

A concentration of OH⁻ in the liquid base catalyst is 0.01 mol/L to 10 mol/L.

Conditions of the reaction are as follows: a reaction temperature is 130°C to 200°C; in the raw materials, a molar ratio of methyl methoxyacetate + methoxyacetic acid to water is 1:8 to 1:3, and a molar ratio of methyl methoxyacetate to methoxyacetic acid is 4:1 to 9:1; and a reaction pressure is 0.1 MPa to 0.3 MPa.

When the reaction zone includes a single fixed-bed reactor or a plurality of fixed-bed reactors connected in series and/or parallel, a WHSV of the methyl methoxyacetate and the methoxyacetic acid in the raw materials is 0.1 h⁻¹ to 3 h⁻¹.

The methyl methoxyacetate, methoxyacetic acid, and water in the raw materials each are a freshly-added raw material and/or an unreacted raw material left after a product is separated.

When the raw materials are allowed to pass through a reaction zone loaded with an acidic molecular sieve catalyst, an inert carrier gas selected from the group consisting of nitrogen and argon is introduced.

Unless otherwise specified, the raw materials in the embodiments of the present application all are purchased from commercial sources.

Analysis methods and conversion rate and selectivity calculation in the embodiments are as follows:
An Agilent7890B gas chromatograph is used to analyze products other than glycolic acid and unreacted raw materials, where an FID detector is connected to a DB-FFAP capillary column and a TCD detector is connected to a Porapak Q packed column. A liquid chromatograph is used to analyze glycolic acid, where a separation column is a C₁₈ column and a detector is an ultraviolet (UV) detector.

In an embodiment of the present application, both a conversion rate and selectivity are calculated based on a mole number of carbon: methyl methoxyacetate conversion rate = [(mole number of carbon in fed methyl methoxyacetate) - (mole number of carbon in discharged methyl methoxyacetate)] ÷ (mole number of carbon in fed methyl methoxyacetate) × 100%; methoxyacetic acid conversion rate = [(mole number of carbon in fed methoxyacetic acid) - (mole number of carbon in discharged methoxyacetic acid)] ÷ (mole number of carbon in fed methoxyacetic acid) × 100%; and selectivity for a product = (mole number of carbon in a discharged product) ÷ (total mole number of carbon in all discharged carbon-containing products) × 100%

The present application will be described in detail below with reference to examples, but the present application is not limited to these examples.

### Catalyst performance test

### Example 1

An acidic H-ZSM-5 molecular sieve with a Si/Al ratio of 20 produced by Zhongke Catalysis New Technology (Dalian) Co., Ltd. was selected, crushed, and sieved to obtain 0.4 mm to 0.8 mm particles; 2 g of the particles was taken and filled into a stainless steel reaction tube with an inner diameter of 8 mm and activated with 50 mL/min nitrogen at 500°C for 4 h; a reaction was conducted for 24 h under the following conditions: a reaction temperature (T) was 160°C, and a reaction pressure (P) was 0.1 MPa; a molar ratio of methyl methoxyacetate + methoxyacetic acid to water was 1:6, and a molar ratio of methyl methoxyacetate to methoxyacetic acid was 5:1; and a WHSV of methyl methoxyacetate and methoxyacetic acid was 0.6 h⁻¹; and after the reaction was completed, products were analyzed by gas chromatography and liquid chromatography. Reaction results based on a mole number of carbon were shown in Table 1. Negative-ion mass spectrometry results of glycolic acid in liquid chromatography-mass spectrometry analysis of products were shown in FIG. 1.

### Examples 2 to 8

The catalyst, reaction conditions, and reaction results were shown in Table 1. Other operations were the same as in Example 1.

**Table 1 Catalytic reaction results in Examples 1 to 8**

| **Ex a m ple** | **Acidic molecular sieve catalyst** | **Manufacturer** | **Si/Al ratio** | **Reaction conditions** | **Methyl methoxya cetate conversio n rate (%)** | **Methox yacetic acid conversi on rate (%)** | **Glycolic acid selectivity (%)** | **Methyl glycolate selectivity (%)** | **Methanol and DME selectivity (%)** |
|---|---|---|---|---|---|---|---|---|---|
| 1 | H-ZSM-5 | Zhongke Catalysis New Technology (Dalian) Co., Ltd. | 20 | T = 160°C; P = 0.1 MPa; WHSV = 0.6 h⁻¹; (methyl methoxyacetate + methoxyacetic acid) : water = 1:6; and (methyl methoxy acetate/methox yacetic acid) = 5:1 | 80.1 | 49.9 | 45.7 | 9.0 | 45.3 |
| 2 | H-Y | Zhongke Catalysis New Technology (Dalian) Co., Ltd. | 3 | T = 160°C; P = 0.1 MPa; WHSV = 0.6 h⁻¹; (methyl methoxyacetate + methoxyacetic acid) : water = 1:6; and (methyl | 57.9 | 28.7 | 44.3 | 8.4 | 47.3 |
| | | | | methoxyacetate/methox yacetic acid) = 5:1 | | | | | |
| 3 | H-ZSM-35 | Catalyst Plant of Nankai University | 50 | T = 160°C; P = 0.1 MPa; WHSV = 0.6 h⁻¹; (methyl methoxyacetate + methoxyacetic acid) : water = 1:6; and (methyl methoxyacetate/methox yacetic acid) = 5:1 | 79.2 | 52.3 | 45.0 | 11.2 | 43.8 |
| 4 | H-β | Zhongke Catalysis New Technology (Dalian) Co., Ltd. | 500 | T = 160°C; P = 0.1 MPa; WHSV = 0.6 h⁻¹; (methyl methoxyacetate + methoxyacetic acid) : water = 1:6; and (methyl methoxyacetate/methox yacetic acid) = 5:1 | 56.2 | 35.9 | 27.7 | 36.0 | 36.3 |
| 5 | H-Mordenite | Yanchang Zhongke (Dalian) Energy Technology Co., Ltd. | 10 | T = 160°C; P = 0.1 MPa; WHSV = 0.6 h⁻¹; (methyl methoxyacetate + methoxyacetic acid) : water = 1:6; and (methyl methoxyacetate/methox yacetic acid) = 5:1 | 55.5 | 29.3 | 35.7 | 24 | 40.3 |
| 6 | H-MCM-22 | Yanchang Zhongke (Dalian) Energy Technology Co., Ltd. | 100 | T = 160°C; P = 0.1 MPa; WHSV = 0.6 h⁻¹; (methyl methoxyacetate + methoxyacetic acid) : water = 1:6; and (methyl methoxyacetate/methox yacetic acid) = 5:1 | 54.3 | 34.2 | 33.7 | 27.0 | 39.3 |
| 7 | H-ZSM-5 | Zhongke Catalysis New Technology (Dalian) Co., Ltd. | 20 | T = 260°C; P = 10 MPa; WHSV = 3.0 h⁻¹; (methyl methoxyacetate + methoxyacetic acid) : water = 1:20; (methyl methoxyacetate/methox yacetic acid) = 4:1; and nitrogen = 100 mL min⁻¹ | 51.0 | 49.7 | 40.3 | 17.4 | 42.3 |
| 8 | H-ZSM-5 | Zhongke Catalysis New Technology (Dalian) Co., Ltd. | 20 | T = 60°C; P = 0.3 MPa; WHSV = 0.1 h⁻¹; (methyl methoxyacetate + methoxyacetic acid) : water = 1:2; and (methyl methoxy acetate/methox yacetic acid) = 4:1 | 24.6 | 31.2 | 42.3 | 16.2 | 41.5 |

It can be seen from Table 1 that the acidic molecular sieve exhibits excellent catalytic performance in hydrolysis of methyl methoxyacetate and methoxyacetic acid to produce glycolic acid and methyl glycolate, and leads to high selectivity for a target product.

### Example 9

Reaction results of different reaction times in Example 9 were shown in Table 2.

**Table 2 Catalytic reaction results in Example 9**

| **Reaction time (h)** | **Methyl methoxyacetate conversion rate (%)** | **Methoxyacetic acid conversion rate (%)** | **Glycolic acid selectivity (%)** | **Methyl glycolate selectivity (%)** |
|---|---|---|---|---|
| 24 | 80.1 | 49.9 | 45.7 | 9.0 |
| 100 | 80.2 | 49.8 | 45.6 | 9.2 |
| 500 | 81.1 | 46.3 | 45.8 | 9.1 |
| 1000 | 78.3 | 47.1 | 45.1 | 8.7 |
| 2000 | 73.3 | 47.1 | 44.8 | 8.2 |
| 4000 | 73.6 | 45.1 | 44.9 | 8.5 |
| 8000 | 73.2 | 44.7 | 44.1 | 9.2 |

It can be seen from Table 2 that the acidic molecular sieve catalyst, especially the H-ZSM-5 molecular sieve catalyst, leads to a high raw material conversion rate during the hydrolysis, and has a long life.

### Example 10

A DB757 strongly-acidic sulfonic acid group-containing exchange resin with an exchange degree of 3.2 mmol/g commercially purchased from Dandong Mingzhu Special Resin Co., Ltd. was used instead of the catalyst in Example 1 and activated with 50 mL/min nitrogen at 100°C for 4 h, and other conditions and operations were the same as in Example 1. Reaction results were shown in Table 3.

### Example 11

γ-alumina with an ammonia adsorption capacity of 0.29 mmol/g commercially purchased from Beijing Yanxin Technology Development Co., Ltd was used instead of the catalyst in Example 10, and other conditions and operations were the same as in Example 10. Reaction results were shown in Table 3.

### Example 12

A 202 FC strongly-alkaline quaternary ammonium group-containing exchange resin with an exchange degree of 3.5 mmol/g commercially purchased from Dandong Mingzhu Special Resin Co., Ltd. was used instead of the catalyst in Example 10, and other conditions and operations were the same as in Example 10. Reaction results were shown in Table 3.

### Example 13

Hydrotalcite with a composition of [Mg_{0.8}Al_{0.2}(OH)₂]⁰²⁺[CO₃²⁻]0.1·2 H₂O was used instead of the catalyst in Example 10, and other conditions and operations were the same as in Example 10. Reaction results were shown in Table 3.

### Example 14

Hydroxyapatite with a composition of Ca₁₀(PO₄)₆ (OH)₂ was used instead of the catalyst in Example 10, and other conditions and operations were the same as in Example 10. Reaction results were shown in Table 3.

**Table 3 Catalytic reaction results in Examples 10 to 14**

| **Example** | **Catalyst** | Methyl methoxyacetate conversion rate (%) | Methoxyacetic acid conversion rate (%) | Glycolic acid selectivity (%) | Methyl glycolate selectivity (%) |
|---|---|---|---|---|---|
| 10 | Strongly-acidic sulfonic acid group-containing exchange resin | 55.3 | 31.2 | 33.7 | 26.3 |
| 11 | γ-alumina | 25.3 | 25.2 | 31.6 | 24.2 |
| 12 | Strongly-alkaline quaternary ammonium group-containing | 27.3 | 28.2 | 33.6 | 26.2 |
| | exchange resin | | | | |
| 13 | Hydrotalcite | 50.3 | 27.2 | 36.5 | 21.3 |
| 14 | Hydroxyapatite | 32.5 | 30.2 | 30.2 | 30.5 |

It can be seen from Table 3 that the solid acid and base catalysts such as a strongly-acidic resin, γ-alumina, an alkaline resin, hydrotalcite, and hydroxyapatite can also catalyze hydrolysis of methyl methoxyacetate and methoxyacetic acid to produce glycolic acid and methyl glycolate.

### Example 15

86.7 g of methyl methoxyacetate, 15 g of methoxyacetic acid, and 108 g of water were added to a reactor, and 10 mL of a 0.1 mol/L sulfuric acid aqueous solution was added as a catalyst; a reaction was conducted for 24 h under the following conditions: reaction temperature: 160°C, reaction pressure: 0.2 MPa, and stirring speed: 300 rpm; and after the reaction was completed, reaction results were shown in Table 4.

**Table 4 Catalytic reaction results in Example 15**

| **Example** | **Catalyst** | Methyl methoxyacetate conversion rate (%) | Methoxyacetic acid conversion rate (%) | Glycolic acid selectivity (%) | Methyl glycolate selectivity (%) |
|---|---|---|---|---|---|
| 15 | Sulfuric acid aqueous solution | 65.3 | 42.2 | 40.8 | 27.9 |
| | | | | | |

It can be seen from Table 4 that the liquid acid can also catalyze hydrolysis of methyl methoxyacetate and methoxyacetic acid to produce glycolic acid and methyl glycolate.

### Example 16

A hydrolysis reaction of methyl methoxyacetate and methoxyacetic acid was tested by a batch catalytic distillation method. A distillation column was a glass column with a diameter of 30 mm, and the distillation column body was filled with an inert annular packing material of 3.0 mm × 3.0 mm, with a packing height of 2.0 m. The distillation column was heated by a heating jacket, and a condenser at a top of the column had a temperature of -15°C.

86.7 g of methyl methoxyacetate, 15 g of methoxyacetic acid, and 108 g of water were added to a reactor, and 10 g of the acidic H-ZSM-5 molecular sieve with a Si/Al ratio of 20 in Example 1 was added as a catalyst; a reaction was conducted for 10 h under the following conditions: reaction temperature: 150°C, reaction pressure: 0.1 MPa, magneton stirring speed: 500 rpm, and reflux ratio: 2; and after the reaction was completed, conversion rates of methyl methoxyacetate and methoxyacetic acid both were about 100%, the selectivity of glycolic acid was 43.5%, and the selectivity of methyl glycolate was 13.0%.

### Example 17

The acidic H-ZSM-5 molecular sieve with a Si/Al ratio of 20 in Example 1 was molded with alumina or silicon oxide into a strip, and after the molding, a content of the alumina or silicon oxide in the molded catalyst was 20 wt%. Other conditions and operations remained unchanged. Reaction results were shown in Table 5.

**Table 5 Catalytic reaction results in Examples 1 and 17**

| **Example** | **Forming agent** | **Methyl methoxyacetate conversion rate (%)** | **Methoxyacetic acid conversion rate (%)** | **Glycolic acid selectivity (%)** | **Methyl glycolate selectivity (%)** |
|---|---|---|---|---|---|
| 1 | None | 80.1 | 49.9 | 45.7 | 9.0 |
| 17 | Alumina | 75.3 | 40.2 | 44.2 | 10.1 |
| 17 | Silicon oxide | 75.6 | 41.8 | 44.9 | 10.3 |

It can be seen from Table 5 that the catalytic activity of the acidic molecular sieve catalyst remains basically unchanged after the molding with alumina or silicon oxide.

### Example 18

A catalyst obtained after 8,000 h of the reaction in Example 9 was treated with 500 mL min⁻¹ of a mixed gas of oxygen and nitrogen in a molar ratio of 5/95 at 600°C for 4 h, and then used in the reaction in Example 9. Reaction results were shown in Table 6.

**Table 6 Catalytic reaction results in Example 18**

| **Reaction time (h)** | **Methyl methoxyacetate conversion rate (%)** | **Methoxyacetic acid conversion rate (%)** | **Glycolic acid selectivity (%)** | **Methyl glycolate selectivity (%)** |
|---|---|---|---|---|
| 24 | 80.3 | 50.2 | 44.7 | 9.7 |
| 100 | 80.0 | 49.7 | 44.8 | 9.6 |
| 500 | 79.5 | 48.8 | 45.3 | 8.9 |

It can be seen from Table 6 that, after being calcined and regenerated with the oxygen/nitrogen mixed gas, the reacted catalyst can be basically restored to reaction performance of a fresh catalyst.

## Claims

1. A method for preparing glycolic acid and methyl glycolate through hydrolysis of methyl methoxyacetate and methoxyacetic acid, comprising: allowing raw materials comprising the methyl methoxyacetate, the methoxyacetic acid, and water to contact and react with a catalyst to produce the glycolic acid and the methyl glycolate,
**characterized in that**
the catalyst is a solid acid catalyst;
the solid acid catalyst is at least one selected from the group consisting of an acidic molecular sieve catalyst, an acidic resin catalyst, and an acidic alumina catalyst; and
the acidic molecular sieve catalyst comprises an acidic molecular sieve.

2. The method according to claim 1, wherein the acidic molecular sieve is at least one selected from the group consisting of an acidic MFI-structured molecular sieve, an acidic FAU-structured molecular sieve, an acidic FER-structured molecular sieve, an acidic BEA-structured molecular sieve, an acidic MOR-structured molecular sieve, and an acidic MWW-structured molecular sieve.

3. The method according to claim 1, wherein the acidic molecular sieve is at least one selected from the group consisting of an acidic ZSM-5 molecular sieve, an acidic Y molecular sieve, an acidic ZSM-35 molecular sieve, an acidic β molecular sieve, an acidic MOR molecular sieve, and an acidic MCM-22 molecular sieve.

4. The method according to claim 1, wherein the acidic molecular sieve is at least one selected from the group consisting of a hydrogen-type ZSM-5 molecular sieve, a hydrogen-type Y molecular sieve, a hydrogen-type ZSM-35 molecular sieve, a hydrogen-type β molecular sieve, a hydrogen-type MOR molecular sieve, and a hydrogen-type MCM-22 molecular sieve.

5. The method according to claim 1, wherein a Si/Al atom ratio of the acidic molecular sieve is 3 to 500.

6. The method according to claim 1, wherein a content of the acidic molecular sieve in the acidic molecular sieve catalyst is 50 wt% to 100 wt%.

7. The method according to claim 1, wherein the acidic molecular sieve catalyst further comprises a forming agent;
the forming agent is an oxide; and
the oxide is one selected from the group consisting of alumina and silicon oxide.

8. The method according to claim 7, wherein a content of the forming agent in the acidic molecular sieve catalyst is m, and 0 < m ≤ 50 wt%.

9. The method according to claim 1, wherein the acidic molecular sieve catalyst is a fresh acidic molecular sieve catalyst and/or a regenerated acidic molecular sieve catalyst; and
the fresh acidic molecular sieve catalyst is an unused acidic molecular sieve catalyst.

10. The method according to claim 9, wherein a regeneration method of the acidic molecular sieve catalyst comprises:
treating an inactivated acidic molecular sieve catalyst with an oxygen-containing regeneration gas at 400°C to 800°C for 0.5 h to 24 h to obtain the regenerated acidic molecular sieve catalyst,
wherein a volume fraction of oxygen in the oxygen-containing regeneration gas is 0.5% to 50%.

11. The method according to claim 1, wherein the acidic resin catalyst is any strongly-acidic cation exchange resin.

12. The method according to claim 11, wherein a skeleton structure in the strongly-acidic cation exchange resin is a copolymer of styrene and divinylbenzene (DVB); and
an acidic group in the strongly-acidic cation exchange resin is a sulfonic acid group.

13. The method according to claim 1, wherein the acidic alumina catalyst is γ-alumina.

14. The method according to claim 1, wherein conditions of the reaction are as follows:
a reaction temperature is 60°C to 260°C;
a reaction pressure is 0.1 MPa to 10 MPa; and
in the raw materials, a ratio of a total mole number of the methyl methoxyacetate and the methoxyacetic acid to a mole number of the water is 1:2 to 1:20.

15. The method according to claim 14, wherein the conditions of the reaction are as follows:
the reaction temperature is 130°C to 200°C;
the reaction pressure is 0.1 MPa to 0.3 MPa; and
in the raw materials, a ratio of a total mole number of the methyl methoxyacetate and the methoxyacetic acid to a mole number of the water is 1:3 to 1:8 and
a molar ratio of the methyl methoxyacetate to the methoxyacetic acid is 4: 1 to 9:1.

## Patentansprüche

1. Verfahren zur Herstellung von Glykolsäure und Methylglykolat durch Hydrolyse von Methylmethoxyacetat und Methoxyessigsäure, umfassend: In Kontakt Bringen und Reagieren lassen von Rohmaterialien, umfassend das Methylmethoxyacetat, die Methoxyessigsäure und Wasser, mit einem Katalysator zur Herstellung von Glykolsäure und Methylglykolat,
**dadurch gekennzeichnet, dass**
der Katalysator ein fester saurer Katalysator ist;
der feste saure Katalysator mindestens einer ist, der aus der Gruppe ausgewählt ist, die aus einem sauren Molekularsiebs-Katalysator, einem sauren Harzkatalysator und einem sauren Aluminiumoxidkatalysator besteht; und
der saure Molekularsiebs-Katalysator ein saures Molekularsieb umfasst.

2. Verfahren nach Anspruch 1, wobei das saure Molekularsieb mindestens eines ist, das aus der Gruppe ausgewählt ist, die aus einem sauren Molekularsieb mit MFI-Struktur, einem sauren Molekularsieb mit FAU-Struktur, einem sauren Molekularsieb mit FER-Struktur, einem sauren Molekularsieb mit BEA-Struktur, einem sauren Molekularsieb mit MOR-Struktur und einem sauren Molekularsieb mit MWW-Struktur besteht.

3. Verfahren nach Anspruch 1, wobei das saure Molekularsieb mindestens eines ist, das aus der Gruppe ausgewählt ist, die aus einem sauren ZSM-5-Molekularsieb, einem sauren Y-Molekularsieb, einem sauren ZSM-35-Molekularsieb, einem sauren β-Molekularsieb, einem sauren MOR-Molekularsieb und einem sauren MCM-22-Molekularsieb besteht.

4. Verfahren nach Anspruch 1, wobei das saure Molekularsieb mindestens eines ist, das aus der Gruppe ausgewählt ist, die aus einem ZSM-5-Molekularsieb vom Wasserstoff-Typ, einem Y-Molekularsieb vom Wasserstoff-Typ, einem ZSM-35-Molekularsieb vom Wasserstoff-Typ, einem β-Molekularsieb vom Wasserstoff-Typ, einem MOR-Molekularsieb vom Wasserstoff-Typ und einem MCM-22-Molekularsieb vom Wasserstoff-Typ besteht.

5. Verfahren nach Anspruch 1, wobei das Si/Al-Atomverhältnis des sauren Molekularsiebs 3 bis 500 beträgt.

6. Verfahren nach Anspruch 1, wobei der Gehalt des sauren Molekularsiebs im sauren Molekularsiebs-Katalysator 50 Gew.-% bis 100 Gew.-% beträgt.

7. Verfahren nach Anspruch 1, wobei der saure Molekularsiebs-Katalysator ferner ein Formungsmittel umfasst;
das Formungsmittel ein Oxid ist; und
das Oxid eines ist, das aus der Gruppe ausgewählt ist, die aus Aluminiumoxid und Siliziumoxid besteht.

8. Verfahren nach Anspruch 7, wobei ein Gehalt des Formungsmittels in dem sauren Molekularsiebs-Katalysator m ist und 0 < m ≤ 50 Gew.-%.

9. Verfahren nach Anspruch 1, wobei der saure Molekularsiebs-Katalysator ein frischer saurer Molekularsiebs-Katalysator und/oder ein regenerierter saurer Molekularsiebs-Katalysator ist; und
der frische saure Molekularsiebs-Katalysator ein unbenutzter saurer Molekularsiebs-Katalysator ist.

10. Verfahren nach Anspruch 9, wobei ein Regenerierungsverfahren des sauren Molekularsiebs-Katalysators umfasst:
behandeln eines inaktivierten sauren Molekularsiebs-Katalysators mit einem sauerstoffhaltigen Regenerierungsgas bei 400 °C bis 800 °C für 0,5 h bis 24 h, um den regenerierten sauren Molekularsiebs-Katalysator zu erhalten,
wobei der Volumenanteil von Sauerstoff in dem sauerstoffhaltigen Regenerationsgas 0,5 % bis 50 % beträgt.

11. Verfahren nach Anspruch 1, wobei der saure Harzkatalysator ein beliebiges stark saures Kationenaustauscherharz ist.

12. Verfahren nach Anspruch 11, wobei eine Gerüststruktur in dem stark sauren Kationenaustauscherharz ein Copolymer aus Styrol und Divinylbenzol (DVB) ist; und
eine saure Gruppe in dem stark sauren Kationenaustauscherharz eine Sulfonsäuregruppe ist.

13. Verfahren nach Anspruch 1, wobei der saure Aluminiumoxidkatalysator γ-Aluminiumoxid ist.

14. Verfahren nach Anspruch 1, wobei die Reaktionsbedingungen wie folgt sind:
eine Reaktionstemperatur beträgt 60 °C bis 260 °C;
ein Reaktionsdruck 0,1 MPa bis 10 Mpa beträgt; und
in den Rohmaterialien das Verhältnis der Gesamtmolzahl von Methylmethoxyacetat und Methoxyessigsäure zur Molzahl von Wasser 1:2 bis 1:20 beträgt.

15. Verfahren nach Anspruch 14, wobei die Reaktionsbedingungen wie folgt sind:
die Reaktionstemperatur beträgt 130 °C bis 200 °C;
der Reaktionsdruck 0,1 MPa bis 0,3 MPa beträgt; und
in den Rohmaterialien das Verhältnis der Gesamtmolzahl von Methylmethoxyacetat und Methoxyessigsäure zur Molzahl des Wassers 1:3 bis 1:8 beträgt und
das Molverhältnis von Methylmethoxyacetat zur Methoxyessigsäure 4:1 bis 9:1 beträgt.

## Revendications

1. Procédé de préparation d'acide glycolique et de glycolate de méthyle par hydrolyse de méthoxyacétate de méthyle et d'acide méthoxyacétique, comprenant : permettre aux matières premières comprenant le méthoxyacétate de méthyle, l'acide méthoxyacétique et de l'eau d'entrer en contact et de réagir avec un catalyseur pour produire l'acide glycolique et le glycolate de méthyle,
**caractérisé en ce que**
le catalyseur est un catalyseur acide solide ;
le catalyseur acide solide est l'un quelconque choisi parmi un catalyseur de tamis moléculaire acide, un catalyseur de résine acide et d'un catalyseur d'alumine acide ; et
le catalyseur de tamis moléculaire acide comprend un tamis moléculaire acide.

2. Procédé selon la revendication 1, dans lequel le tamis moléculaire acide est au moins l'un choisi parmi un tamis moléculaire à structure MFI acide, un tamis moléculaire à structure FAU acide, un tamis moléculaire à structure FER acide, un tamis moléculaire à structure BEA acide, un tamis moléculaire à structure MOR acide et un tamis moléculaire à structure MWW acide.

3. Procédé selon la revendication 1, dans lequel le tamis moléculaire acide est au moins l'un choisi parmi un tamis moléculaire ZSM-5 acide, un tamis moléculaire Y acide, un tamis moléculaire ZSM-35 acide, un tamis moléculaire β acide, un tamis moléculaire MOR acide et un tamis moléculaire MCM-22 acide.

4. Procédé selon la revendication 1, dans lequel le tamis moléculaire acide est au moins l'un choisi parmi un tamis moléculaire ZSM-5 de type hydrogène, un tamis moléculaire Y de type hydrogène, un tamis moléculaire ZSM-35 de type hydrogène, un tamis moléculaire β de type hydrogène, un tamis moléculaire MOR de type hydrogène et un tamis moléculaire MCM-22 de type hydrogène.

5. Procédé selon la revendication 1, dans lequel un rapport atomique Si/Al du tamis moléculaire acide est de 3 à 500.

6. Procédé selon la revendication 1, dans lequel une teneur du tamis moléculaire acide dans le catalyseur de tamis moléculaire acide est de 50% en poids à 100% en poids.

7. Procédé selon la revendication 1, dans lequel le catalyseur de tamis moléculaire acide comprend en outre un agent de formation ;
l'agent de formation est un oxyde ; et
l'oxyde est choisi parmi l'alumine et l'oxyde de silicium.

8. Procédé selon la revendication 7, dans lequel une teneur de l'agent de formation dans le catalyseur de tamis moléculaire acide est m, et 0 < m ≤ 50% en poids.

9. Procédé selon la revendication 1, dans lequel le catalyseur de tamis moléculaire acide est un catalyseur de tamis moléculaire acide frais et/ou un catalyseur de tamis moléculaire acide régénéré ; et
le catalyseur de tamis moléculaire acide frais est un catalyseur de tamis moléculaire acide non utilisé.

10. Procédé selon la revendication 9, dans lequel un procédé de régénération du catalyseur de tamis moléculaire acide comprend :
le traitement d'un catalyseur de tamis moléculaire acide non-activé avec un gaz de régénération contenant de l'oxygène à 400°C à 800°C pendant 0,5 h à 24 h pour obtenir le catalyseur de tamis moléculaire acide régénéré,
dans lequel une fraction volumique d'oxygène dans le gaz de régénération contenant de l'oxygène est de 0,5% à 50%.

11. Procédé selon la revendication 1, dans lequel le catalyseur de résine acide est n'importe quelle résine échangeuse de cations fortement acide.

12. Procédé selon la revendication 11, dans lequel une structure de squelette dans la résine échangeuse de cations fortement acide est un copolymère de styrène et de divinylbenzène (DVB) ; et
un groupe acide dans la résine échangeuse de cations fortement acide est un groupe acide sulfonique.

13. Procédé selon la revendication 1, dans lequel le catalyseur d'alumine acide est la γ-alumine.

14. Procédé selon la revendication 1, dans lequel les conditions de la réaction sont les suivantes :
une température de réaction est de 60°C à 260°C ;
une pression de réaction est de 0,1 MPa à 10 MPa ; et
dans les matières premières, un rapport d'un nombre de moles total du méthoxyacétate de méthyle et de l'acide méthoxyacétique à un nombre de moles de l'eau est de 1:2 à 1:20.

15. Procédé selon la revendication 14, dans lequel les conditions de la réaction sont les suivantes :
la température de réaction est de 130°C à 200°C ;
la pression de réaction est de 0,1 MPa à 0,3 MPa ; et
dans les matières premières, un rapport d'un nombre de moles total du méthoxyacétate de méthyle et de l'acide méthoxyacétique à un nombre de moles de l'eau est de 1:3 à 1:8 et
un rapport molaire du méthoxyacétate de méthyle à l'acide méthoxyacétique est de 4:1 à 9:1.
